# EUROPEAN PATENT APPLICATION

(11) **EP 4 482 082 A1**
(43) Date of publication of application: **25.12.2024**
(21) Application number: 23180778.5
(22) Date of filing: 21.06.2023
(51) Int. Cl.: H04L 9/40, G06Q 10/30, G06Q 10/0833, G06Q 50/04, H04L 9/00, G06Q 10/06, G06Q 10/087, G06Q 30/018, G06Q 50/28

(54) **ENVIRONMENTAL ATTRIBUTES FOR ACTIVE PHARMACEUTICAL INGREDIENTS AND/OR INTERMEDIATES THEREOF**

(71) Applicant: BASF SE, 67056 Ludwigshafen am Rhein (DE)
(72) Inventor: Grumbrecht, Bastian, 67061 Ludwigshafen (DE); Klosterhalfen, Steffen, 67061 Ludwigshafen (DE); Krueger, Christian, 67056 Ludwigshafen (DE); Wenzl, Kurt, 67061 Ludwigshafen (DE); Alba Perez, Ana, 67056 Ludwigshafen (DE); Anderlohr, Christopher Alec, 67056 Ludwigshafen (DE); Binder, Martin, 67061 Ludwigshafen (DE); Pistillo, Alessandro, 67056 Ludwigshafen (DE)
(74) Representative: BASF IP Association

(57) **Abstract**

Disclosed are systems for producing an active pharmaceutical ingredient and/or intermediate(s) thereof associated with a digital asset, methods for producing an active pharmaceutical ingredient and/or intermediate(s) thereof associated with a digital asset, apparatuses for generating a digital asset, computer-implemented methods for generating a chemical passport, computer program elements for generating a digital asset, uses of an active pharmaceutical ingredient and/or intermediate(s) thereof associated with a digital asset, uses of a digital asset, products produced from the active pharmaceutical ingredient and/or intermediate(s) thereof and associated with a digital asset, a digital asset including one or more decentral identifier(s) and data related to the environmental impact data, apparatuses for producing a product associated with the digital asset and methods for producing a product associated with the digital asset.

## Description

### TECHNICAL FIELD

The present disclosure relates to systems for producing an active pharmaceutical ingredient and/or intermediate(s) thereof associated with an active pharmaceutical ingredient and/or intermediate(s) thereof passport or digital asset, methods for producing an active pharmaceutical ingredient and/or intermediate(s) thereof associated with an active pharmaceutical ingredient and/or intermediate(s) thereof passport or digital asset, apparatuses for generating a passport or digital asset associated with an active pharmaceutical ingredient and/or intermediate(s) thereof, computer-implemented methods for generating an active pharmaceutical ingredient and/or intermediate(s) thereof passport or digital asset, computer program elements for generating an active pharmaceutical ingredient and/or intermediate(s) thereof passport or digital asset, uses of an active pharmaceutical ingredient and/or intermediate(s) thereof associated with an active pharmaceutical ingredient and/or intermediate(s) thereof passport or digital asset, uses of an active pharmaceutical ingredient and/or intermediate(s) thereof passport or digital asset, products produced from the active pharmaceutical ingredient and/or intermediate(s) thereof and associated with an active pharmaceutical ingredient and/or intermediate(s) thereof passport or digital asset, an active pharmaceutical ingredient and/or intermediate(s) thereof, an active pharmaceutical ingredient and/or intermediate(s) thereof passport or digital asset including one or more decentral identifier(s) and data related to environmental impact data, apparatuses for producing a product associated with the active pharmaceutical ingredient and/or intermediate(s) thereof passport or digital asset and methods for producing a product associated with the active pharmaceutical ingredient and/or intermediate(s) thereof passport or digital asset.

### TECHNICAL BACKGROUND

In supply chains the environmental impact of each supply chain participants is of great interest. Specifically in the field of chemistry, active pharmaceutical ingredients and/or intermediates thereof are employed for a wide range of applications and are supplied to diverse value chains. In such complex systems transparency between value chain participants is hard to achieve.

### SUMMARY OF THE INVENTION

In one aspect disclosed is a system for producing an active pharmaceutical ingredient and/or intermediate(s) thereof associated with an active pharmaceutical ingredient and/or intermediate(s) thereof passport or a digital asset, the system comprising:
- a chemical production network configured to produce the active pharmaceutical ingredient and/or intermediate(s) thereof wherein the active pharmaceutical ingredient and/or intermediate(s) thereof is produced from one or more input material(s) through one or more chemical process(s) of the chemical production network, wherein the one or more input material(s) and/or the one or more chemical process(s) are associated with environmental attribute(s);
- a production operating apparatus configured to generate the active pharmaceutical ingredient and/ or intermediate(s) thereof passport or digital asset by
   providing a decentral identifier associated with the produced active pharmaceutical ingredient and/ or intermediates thereof and the one or more environmental attribute(s) of the one or more input material(s) and/or the one or more chemical process(es);
   linking the decentral identifier and the environmental attribute(s);
- optionally the chemical production network or system configured to provide the produced active pharmaceutical ingredient and/ or intermediate(s) thereof in association with the active pharmaceutical ingredient and/or intermediate(s) thereof passport or digital asset.

In another aspect disclosed is a system for producing an active pharmaceutical ingredient and/or intermediate(s) thereof associated with an active pharmaceutical ingredient and/or intermediate(s) thereof passport or digital asset, the system comprising:
- a chemical production network configured to produce the active pharmaceutical ingredient and/or intermediate(s) thereof from one or more input material(s) through chemical process(s), wherein the one or more input material(s) and/or the chemical process(s) are associated with environmental attribute(s);
- a production operating apparatus configured to generate the active pharmaceutical ingredient and/or intermediate(s) thereof passport or digital asset by providing and/or linking a decentral identifier associated with the active pharmaceutical ingredient and/or intermediate(s) thereof and one or more environmental attribute(s) associated with the one or more input material(s) and/or the chemical process(s).

In another aspect disclosed is a method for producing an active pharmaceutical ingredient and/or intermediate(s) thereof associated with an active pharmaceutical ingredient and/or intermediate(s) thereof passport or digital asset, wherein the method comprises:
- producing the active pharmaceutical ingredient and/or intermediate(s) thereof from one or more input material(s) through one or more chemical process(s) of a chemical production network, wherein the one or more input material(s) and/or the one or more chemical process(s) are associated with environmental attribute(s);
- generating the active pharmaceutical ingredient and/or intermediate(s) thereof passport or digital asset by
   providing a decentral identifier associated with the produced active pharmaceutical ingredient and/ or intermediate(s) thereof and one or more environmental attribute(s) associated with the one or more input material(s) and/or the one or more chemical process(es);
   linking the decentral identifier and the one or more environmental attribute(s);
- providing the produced active pharmaceutical ingredients and/or intermediate(s) thereof in association with the active pharmaceutical ingredient and/or intermediate(s) thereof passport or digital asset.

In another aspect disclosed is a method for producing an active pharmaceutical ingredient and/or intermediate(s) thereof associated with an active pharmaceutical ingredient and/or intermediate(s) thereof passport or a digital asset, wherein the method comprises:
- producing the active pharmaceutical ingredient and/or intermediate(s) thereof from one or more input material(s) through one or more chemical process(s) of a chemical production network, wherein the one or more input material(s) and/or the one or more chemical process(s) are associated with environmental attribute(s),
- generating the active pharmaceutical ingredient and/or intermediate(s) thereof passport or digital asset by providing and/or linking a decentral identifier associated with the active pharmaceutical ingredient and/or intermediate(s) thereof and one or more environmental attribute(s) of the one or more input material(s) and/or the chemical process(s).

In another aspect disclosed is an apparatus for generating a passport or digital asset associated with an active pharmaceutical ingredient and/or intermediate(s) thereof, wherein the active pharmaceutical ingredients and/or intermediates thereof is produced from one or more input material(s) through one or more chemical process(s) of a chemical production network, wherein the one or more input material(s) and/or the one or more chemical process(s) are associated with environmental attribute(s), the apparatus comprising:
- a decentral identity provider configured to provide a decentral identifier associated with the produced active pharmaceutical ingredient and/or intermediate(s) thereof and one or more environmental attribute(s) of the one or more input material(s) and/or the one or more chemical process(s) used to produce the active pharmaceutical ingredient and/or intermediate(s) thereof;
- an assignor configured the decentral identifier and the environmental attribute(s);
- a passport provider configured to provide the active pharmaceutical ingredient and/or intermediate(s) thereof passport or digital asset in association with the produced active pharmaceutical ingredient and/or intermediate(s) thereof to a decentral network, wherein the environmental attribute(s) associated with the active pharmaceutical ingredient and/or intermediate(s) thereof is made accessible to a consumer of the active pharmaceutical ingredient and/or intermediate(s) thereof through the active pharmaceutical ingredient and/or intermediate(s) thereof passport or digital asset.

In another aspect disclosed is a method, e.g. a computer-implemented method, for generating an active pharmaceutical ingredient and/ or intermediate(s) thereof passport or digital asset associated with an active pharmaceutical ingredient and/or intermediate(s) thereof, wherein the active pharmaceutical ingredient and/or intermediate(s) thereof is produced from one or more input material(s) through one or more chemical process(s) of a chemical production network, wherein the one or more input material(s) and/or the one or more chemical process(s) are associated with environmental attribute(s), the method comprising:
- providing a decentral identifier associated with the produced active pharmaceutical ingredient and/or intermediate(s) thereof and one or more environmental attribute(s) of the one or more input material(s) and/or the one or more chemical process(s) used to produce the active pharmaceutical ingredients and/or intermediates thereof;
- linking the decentral identifier and the environmental attribute(s);
- providing the active pharmaceutical ingredient and/or intermediate(s) thereof passport or digital asset in association with the produced active pharmaceutical ingredient and/or intermediate(s) thereof to a decentral network, wherein the environmental attribute(s) associated with the active pharmaceutical ingredient and/or intermediate(s) thereof is made accessible to a user of the active pharmaceutical ingredient and/or intermediate(s) thereof through the active pharmaceutical ingredient and/or intermediate(s) thereof passport or digital asset.

In another aspect disclosed is a computer element, such as a computer readable storage medium, a computer program or a computer program product, comprising instructions, which when executed by a computing node or a computing system, direct the computing node or computing system to carry out the steps of the computer-implemented methods disclosed herein.

In another aspect disclosed is a computer element, such as a computer readable storage medium, a computer program or a computer program product, comprising instructions, which when executed by the apparatuses disclosed herein, direct the apparatuses to carry out steps the apparatuses disclosed herein are configured to execute.

In another aspect disclosed is an active pharmaceutical ingredient and/or intermediate(s) thereof associated with an active pharmaceutical ingredient and/or intermediate(s) thereof passport or a digital asset as produced according to the methods disclosed herein. In another aspect disclosed is an active pharmaceutical ingredient and/or intermediate(s) thereof associated with an active pharmaceutical ingredient and/or intermediate(s) thereof passport or digital asset as produced according to the systems disclosed herein.

In another aspect disclosed is an active pharmaceutical ingredient and/or intermediate(s) thereof 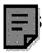 associated with an active pharmaceutical ingredient and/or intermediate(s) thereof passport or digital asset, wherein the active pharmaceutical ingredient and/or intermediate(s) thereof is produced from one or more input material(s) through one or more chemical process(s) of a chemical production network, wherein the one or more input material(s) and/or the one or more chemical process(s) are associated with environmental attribute(s), wherein the active pharmaceutical ingredient and/or intermediate(s) thereof passport or digital asset includes a decentral identifier associated with the produced active pharmaceutical ingredient and/or intermediate(s) thereof and a link to environmental attribute(s) associated with one or more environmental attribute(s) of one or more input material(s) and/or one or more chemical process(s) used to produce the active pharmaceutical ingredient and/or intermediate(s) thereof.

In another aspect disclosed is an active pharmaceutical ingredient and/or intermediate(s) thereof passport or digital asset as generated according to the methods disclosed herein. In another aspect disclosed is an active pharmaceutical ingredient and/or intermediate(s) thereof passport or digital asset as generated according to the apparatuses disclosed herein.

In another aspect disclosed is a production system for producing a product from the active pharmaceutical ingredient and/or intermediate(s) thereof associated with the active pharmaceutical ingredient and/or intermediate(s) thereof passport or digital asset as provided according to the systems, apparatuses or methods disclosed herein. In another aspect disclosed is a production method for producing a product from the active pharmaceutical ingredient and/or intermediate(s) thereof associated with the active pharmaceutical ingredient and/or intermediate(s) thereof passport or digital asset as provided according to the systems, apparatuses or methods disclosed herein.

In another aspect disclosed is a use of the active pharmaceutical ingredient and/or intermediate(s) thereof associated with the active pharmaceutical ingredient and/or intermediate(s) thereof passport or digital asset as disclosed herein for producing a product from the active pharmaceutical ingredient and/or intermediate(s) thereof associated with the active pharmaceutical ingredient and/or intermediate(s) thereof passport or digital asset.

In another aspect disclosed is a use of the active pharmaceutical ingredient and/ or intermediate(s) thereof passport or digital asset as disclosed herein for generating a product passport or digital asset associated with a product produced from the active pharmaceutical ingredient and/or intermediate(s) thereof associated with the active pharmaceutical ingredient and/or intermediate(s) thereof passport or digital asset. In another aspect disclosed is a method for using the digital asset generated according to the methods disclosed herein in the production of a product produced from the active pharmaceutical ingredient and/or intermediate(s) thereof associated with the active pharmaceutical ingredient and/or intermediate(s) thereof passport or digital asset.

In another aspect disclosed is an active pharmaceutical ingredient and/ or intermediate(s) thereof associated with a digital asset including a decentral identifier associated with the active pharmaceutical ingredient and/or intermediate(s) thereof and linked to one or more environmental attribute(s) of the one or more input material(s) and/or the one or more chemical process(s) used to produce the active pharmaceutical ingredient and/or intermediate(s) thereof.

In another aspect disclosed is a use of the active pharmaceutical ingredient and/or intermediate(s) thereof associated with the active pharmaceutical ingredient and/or intermediate(s) thereof passport or digital asset for producing a product from the active pharmaceutical ingredient and/or intermediate(s) thereof and associating the active pharmaceutical ingredient and/or intermediate(s) thereof passport or digital asset with the product produced from the active pharmaceutical ingredient and/or intermediate(s) thereof. In another aspect disclosed is a use of the active pharmaceutical ingredient and/or intermediate(s) thereof associated with the active pharmaceutical ingredient and/or intermediate(s) thereof passport or digital asset for producing a product from the active pharmaceutical ingredient and/or intermediate(s) thereof and deriving a product passport or digital asset from the active pharmaceutical ingredient and/or intermediate(s) thereof passport or digital asset. In another aspect disclosed is a method for using the active pharmaceutical ingredient and/or intermediate(s) thereof associated with the digital asset for producing a product from the active pharmaceutical ingredient and/or intermediate(s) thereof as disclosed herein and deriving a digital asset associated with the product from the active pharmaceutical ingredient and/or intermediate(s) thereof passport or digital asset.

Any disclosure and embodiments described herein relate to the methods, the systems, chemical products, active pharmaceutical ingredients and/or intermediates thereof, active pharmaceutical ingredient and/or intermediate(s) thereof passports or digital assets and the computer elements lined out above and below. Advantageously, the benefits provided by any of the embodiments and examples equally apply to all other embodiments and examples.

### EMBODIMENTS

In the following, embodiments of the present disclosure will be outlined by ways of embodiments and/or example. It is to be understood that the present disclosure is not limited to said embodiments and/or examples.

Determining, generating includes initiating or causing to determine, generate. Providing includes "initiating or causing to access, determine, generate, send or receive". "Initiating or causing to perform an action" includes any processing signal that triggers a computing node to perform the respective action.

The methods, the systems, active pharmaceutical ingredients and/or intermediates thereof, active pharmaceutical ingredient and/or intermediate(s) thereof passports or digital assets and the computer elements disclosed herein provide an efficient, secure and robust way for sharing or exchanging environmental impact data across different participant nodes in value chains. In particular, by providing active pharmaceutical ingredients and/or intermediates thereof specific data via the active pharmaceutical ingredient and/or intermediate(s) thereof passport or digital asset, environmental impacts can be shared and made transparent from the active pharmaceutical ingredient and/or intermediate(s) thereof to the product produced from such active pharmaceutical ingredient and/or intermediate(s) thereof. The active pharmaceutical ingredient and/or intermediate(s) thereof passport or digital asset enables secure data exchange, since data access can be controlled by the active pharmaceutical ingredient and/or intermediate(s) thereof provider, e.g. the entity providing the active pharmaceutical ingredient and/or intermediate(s) thereof. The exchanged data assets can be specific to the active pharmaceutical ingredient and/or intermediate(s) thereof as produced and tailored to the needs of the customer and/or consumer of the active pharmaceutical ingredient and/ or intermediate(s) thereof. This way an improved tracking and tracing of active pharmaceutical ingredient and/or intermediate(s) thereof can be achieved by securely providing environmental impact data in diverse and highly complex value chains. The environmental impact of active pharmaceutical ingredients and/or intermediate(s) thereof can hence be tracked leading to simpler, more efficient and sustainable handling of active pharmaceutical ingredients and/or intermediates thereof by value chain participants.

Active pharmaceutical ingredients are substances that provide pharmacological activity or other direct effect in the diagnosis, cure, mitigation, treatment, or prevention of disease, or to affect the structure or any function of the body. Intermediates thereof are isolated products that are generated during a multi-step route of synthesis of an active pharmaceutical ingredient-

Active pharmaceutical ingredients and/or intermediates thereof associated with the respective active pharmaceutical ingredient and/or intermediate(s) thereof passport or digital asset include, without being limited to, substances that affect hyperpigmentation, rosacea (azelaic acid), wound healing (dexpanthenol), or act as antiseptics, disinfectants (poly[(2-oxopyrrolidin-1yl)ethylene]iodine), topical analgesics, local anesthetic (L-menthol) and non-steroidal antiinflammatory agents (ibuprofen, Ibuprofen sodium dihydrate, racemic ibuprofen lysinate) or intermediates to manufacture certain active ingredients such as 4-hydroxyacetophenone or 2-hydroxyacetophenone.

Active pharmaceutical ingredients and/or intermediates thereof may for example be selected from the following list:

| **Chemical Name** | **Generic or Trade Name(s)** | **CAS No.** |
|---|---|---|
| Azelaic Acid | Dermaz^{®} 99 | 123-99-9 |
| (R)-2,4-Dihydroxy-N-(3-hydroxypropyl)-3,3-dimethylbutanamide | Dexpanthenol | 81-13-0 |
| (RS)-2-(4-isobutylphenyl)-propionic acid | Ibuprofen | 15687-27-1 |
| Sodium-(RS)-2-(4-isobutylphenyl)-propionate dihydrate | Ibuprofen Sodium Dihydrate | 31121-93-4 |
| (±)-2-[4-(2-Methylpropyl)phenyl]propanoic acid lysinate | Racemic Ibuprofen Lysinate | 57469-76-8 |
| 2-Isopropyl-5-methylcyclohexanol, 5-methyl-2-(propan-2-yl)-cyclohexan-1-ol, (1R,2S,5R)- 5-methyl-2-(1-methylethyl)-cyclohexanol | L-menthol | 2216-51-5 |
| Poly[(2-oxopyrrolidin-1 yl)ethylene]iodine | PVP-lodine | 25655-41-8 |
| 2-Hydroxyacetophenon | 2-Hydroxyacetophenon | 118-93-4 |
| 2-Hydroxyacetophenon | 4-Hydroxyacetophenon | 99-93-4 |

The chemical production network may include one or more one or more chemical and/or mechanical process(es). The chemical production network may produce one or more output material(s) through chemical and/or mechanical processing. The chemical production network may include multiple types of production processes for producing one or more output material(s) from one or more input material(s). The chemical production network may produce one or more output material(s) from input material(s) provided to the chemical production network. The chemical production network may include a complex production network producing multiple chemical products via multiple production process(es). The chemical production network may include connected, interconnected and/or non-connected production process(es). The chemical production network may include a composite or Verbund network.

The chemical production network may include identity preserving or segregated production process(es). Identity preserving or segregated in this context may refer to environmental attribute(s) of input material(s) being preserved or segregated in the production process(es). Examples are non-fossil, e.g. renewable or recycled, input materials used to produce the one or more active pharmaceutical ingredients and/or intermediate(s) thereof without fossil content. Further examples are fossil input material(s) used to produce the one or more active pharmaceutical ingredients and/or intermediate(s) thereof with fossil content. Chemical production networks may include non-identity preserving or non-segregated production process(es). Non-identity preserving or non-segregated in this context may refer to non-fossil input material(s) being mixed with fossil input material(s) to produce the active pharmaceutical ingredient and/ or intermediate(s) thereof. For example, fossil and renewable input materials may be mixed to produce an active pharmaceutical ingredient and/or intermediate(s) thereof with fossil and renewable content.

The chemical production network may include one or more production process(es) with multiple production steps. The production steps included in the chemical network may be defined by the physical system boundary of the chemical production network. The system boundary may be defined by location and/or control over production processes or steps. The system boundary may be defined by a site of the chemical production network. The system boundary may be defined by production process(es) or step(s) controlled by one entity or multiple entities jointly. The system boundary may be defined by the value chain with staggered production process(es) or step(s) to the chemical end product, which may be controlled by multiple entities jointly or separately. The chemical production network may include a waste collection, a sorting step, a recycling step such as pyrolysis, a cracking step such as steam cracking, a separation step to separate intermediates of one process step and further processing steps to convert such intermediates to output material(s), in particular the produced active pharmaceutical ingredient and/or intermediates thereof leaving the system boundary of the chemical production network. The input material(s) may enter the physical system boundary of the chemical production network. The entry point(s) of the chemical production network may be marked by the entry of input material(s) to the chemical production network or the system boundary of the chemical network. The output material(s), in particular the produced active pharmaceutical ingredients and/or intermediate(s) thereof, may leave the physical system boundary of the chemical production network. The exit point(s) of the chemical production network may be marked by the exit of output material(s), in particular the produced active pharmaceutical ingredients and/or intermediates thereof, from the chemical production network or the system boundary of the chemical network.

The chemical production network may include one or more production chain(s) for the production of active pharmaceutical ingredients and/or intermediates thereof. The production chain(s) for the production of active pharmaceutical ingredients and/or intermediates thereof may be interconnected. The production chain(s) for the production of active pharmaceutical ingredients and/or intermediates thereof may be interconnected with production chain(s) for the production of other output material(s). The production chain(s) for the production of active pharmaceutical ingredients and/or intermediates thereof may include production chain(s) for the production of intermediates used to produce active pharmaceutical ingredients and/or intermediates thereof. The production chain(s) for the production of active pharmaceutical ingredients and/or intermediates thereof may use input material(s) provided by chemical network(s) for the production of intermediates usable to produce active pharmaceutical ingredients and/or intermediates thereof.

One or more input material(s) may be provided to the chemical production network for producing one or more output material(s), in particular active pharmaceutical ingredient(s) and/or intermediate(s) thereof. The output material(s), in particular active pharmaceutical ingredient(s) and/or intermediate(s) thereof, may be produced from one or more input material(s) through one or more chemical process(s) of the chemical production network. The input material may comprise any input material entering the chemical production network at any entry point. The input material may include organic or inorganic input material(s). The input material may be a precursor product, an intermediate material, a feedstock or a raw material used to produce one or more output material(s), in particular active pharmaceutical ingredient(s) and/or intermediates thereof. The input material may be fed to the chemical production network to produce one or more output material(s), in particular active pharmaceutical ingredient(s) and/or intermediate(s) thereof. The input material may be fed to chemical production network including one or more production process(es) with multiple process steps. The input material may be fed to the chemical production network at the start of the production process or at any intermediate stage of the production process. The input materials entering the chemical production network may be used to produce one or more output material(s), in particular active pharmaceutical ingredient(s) and/ or intermediate(s) thereof.

The input material may be associated with an input material identifier. The input material identifier may comprise any identifier uniquely associated with the input material. The input material identifier may be associated with the physical entity of the input material. The input material identifier may be associated with a single batch of input material. The input material identifier may be associated with a group of input materials. The identifier may be associated with multiple physical entities of the input material. The input material identifier may be associated with continuous or semi-continuous stream of input material. The input material identifier may be associated with a stream of the input material e.g. over a certain time period or from a certain supplier. The input material identifier may be linked or connected to one or more environmental attribute(s).

Environmental attribute may refer to a property related to the environmental impact. Such property may be the property of input material(s), chemical process(es), chemical production network(s) and/or active pharmaceutical ingredient(s) and/or intermediate(s) thereof. The environmental attribute may indicate an environmental performance of input material(s), chemical process(es), chemical production network(s) and/or active pharmaceutical ingredient(s) and/or intermediate(s) thereof. The environmental attribute may be derived from properties of input material(s), chemical process(es), chemical production network(s) and/or active pharmaceutical ingredient(s) and/or intermediate(s) thereof. The environmental attribute may be associated with the environmental impact of input material(s), chemical process(es), chemical production network(s) and/or active pharmaceutical ingredient(s) and/or intermediate(s) thereof at any stage of the lifecycle of the input material(s) and/or active pharmaceutical ingredient(s) and/or intermediate(s) thereof. The stages may include providing raw material, providing feedstock, producing chemical products, such as intermediate products or end products, producing discrete products by using the chemical products, using chemical products or discrete products, treating end-of-life products, recycling end-of-life products, disposing end-of-life products, reusing components from end-of-life products or any subset of stages. The environmental attribute may be specified or may be derived from any activity of one or more entities participating at any stage of the lifecycle of one or more material(s) or product(s).

The environmental attribute may include one or more characteristic(s) that are attributable to environmental impact of input material(s), chemical process(es), chemical production network(s) and/or active pharmaceutical ingredient(s) and/or intermediate(s) thereof. The environmental attribute may include environmental, technical, recyclability or circularity characteristics(s) associated with the environmental impact of input material(s), chemical process(es), chemical production network(s) and/or active pharmaceutical ingredient(s) and/or intermediate(s) thereof.

The environmental attribute may include one or more characteristic(s) that are attributable to the environmental impact of input material(s), chemical process(es), chemical production network(s) and/or active pharmaceutical ingredient(s) and/or intermediate(s) thereof. The environmental attribute may include environmental, technical, recyclability or circularity characteristics(s) associated with the environmental impact of input material(s), chemical process(es), chemical production network(s) and/or active pharmaceutical ingredient(s) and/or intermediate(s) thereof. The one or more environmental attribute(s) may be attributable to the environmental impact of the active pharmaceutical ingredient(s) and/or intermediate(s) thereof. The one or more environmental attribute(s) may relate to environmental, technical, recyclability, circularity and/or complementary risk characteristic(s) of the active pharmaceutical ingredient(s) and/or intermediate(s) thereof.

Environmental characteristic(s) may specify or quantify ecological criteria associated with environmental impact. Environmental characteristic(s) may be or may be derived from measurements taken during the lifecycle. Environmental characteristics may be determined at any stage of the lifecycle and may characterize the environmental impact for such stage or up to such stage. Environmental characteristic(s) may for example include carbon footprint, greenhouse gas emissions, resource usage, air emissions, ozone depletion potential, water pollution, noise pollution, energy consumption, waste reduction, or eutrophication potential. Environmental characteristic(s) may for example include product characteristics related to the production of the product like bio based, vegetable based, animal based, halogen-free, fluorine-free, vegan, halal, kosher, palm oil-free, natural, tox-fee, volatile organic compounds-free or any combinations thereof.

Technical characteristic(s) may specify or quantify performance at least indirectly associated with the environmental impact. Technical characteristic(s) may be or may be derived from measurements taken during the lifecycle. Technical characteristics may be determined at any stage of the lifecycle and may characterize the performance for such stage or up to such stage. Technical characteristic(s) may for example include chemical composition data, raw material composition such as bio-based or recycled input material content specifying e.g. x% non fossil and y% fossil content, bill of materials, product specification data such as product purity, product form (as indication to their impact on dust formation/release), safety data, product extractability, migration data, toxicological data or ecotoxicological data, product component data, safety data, application property data, application instructions, quality data or any combinations thereof.

Circularity characteristic(s) may specify or quantify the life cycle characteristics associated with circular uses. Circularity characteristic(s) may be or may be derived from measurements taken during the lifecycle. Circularity characteristic(s) may be or may be derived from circular data recorded in one or more prior lifecycle(s) including reuse. Circularity characteristic(s) may be determined at any stage of the lifecycle and may characterize the reuse or recycling performance for such stage or up to such stage. Circularity characteristic(s) may for example include recycling data, reuse rate, recycling rate, recycling loops, reuse performance, reused quality or any combinations thereof.

Recyclability characteristic(s) may specify or quantify life cycle characteristics associated with recycling uses. Recyclability characteristic(s) may include the composition of the material including specifically tailored constituents making the material suitable for recycling. Recyclability characteristic(s) may be or may be derived from measurements taken during the lifecycle. Recyclability characteristic(s) may be or may be derived from recycling data recorded in one or more prior lifecycle(s). Recyclability characteristics may be determined at any stage of the lifecycle and may characterize the recycling performance for such stage or up to such stage. Recyclability characteristic(s) may for example include recycling data, number of reuses, recyclate composition, recyclate quality, waste stream composition, waste stream quality or any combinations thereof.

In one embodiment the active pharmaceutical ingredient and/or intermediate(s) thereof passport or digital asset associated with the active pharmaceutical ingredient and/ or intermediate(s) thereof may include mass balanced environmental attributes related to the input material. Mass balanced environmental attributes may include environmental attributes of the input material(s) used to produce the active pharmaceutical ingredient and/ or intermediate(s) thereof, which are tracked and by mass attributable to the active pharmaceutical ingredient and/or intermediate(s) thereof. The environmental impact of input material(s) may be determined based on input material(s) used in the chemical process(s) to produce the active pharmaceutical ingredient and/or intermediate(s) thereof. For example, bio-based, renewable and/or recycled content of input material(s) used to produce the active pharmaceutical ingredient and or intermediate(s) thereof may be tracked. Further for example, biomethane content of input material(s) used to produce the active pharmaceutical ingredient and/or intermediate(s) thereof may be tracked.

Further for example, properties related to the environmental impact of the input material include biomethane may be tracked. Further for example, properties of the chemical process(es) used to produce the active pharmaceutical ingredient and/or intermediate(s) thereof may be tracked. Examples of tracked process properties related to the environmental impact include water consumption, CO₂ emissions and/or Greenhouse Gas (GHG) emissions, amount of waste generation, mixed material generation, design for recycling, energy consumption, processing properties such as less waste or less loss of properties. The properties may be tracked based on a certificate from a certifying agency. The properties may be tracked based on inherent physical properties derived from measurements.

In one embodiment the produced active pharmaceutical ingredient and/or intermediate(s) thereof is connected to the decentral identifier physically identifying the produced active pharmaceutical ingredient and/or intermediate(s) thereof. The production operating apparatus may be configured to provide the decentral identifier associated with a physical entity of the produced active pharmaceutical ingredient and/or intermediate(s) thereof. The production operating apparatus may be configured to link the decentral identifier to a physical identifier of the produced active pharmaceutical ingredient and/or intermediate(s) thereof. The production operating apparatus may be configured to assign the decentral identifier to the physical identifier connected to the produced active pharmaceutical ingredient and/or intermediate(s) thereof. The production operating apparatus may be configured to assign the decentral identifier to the physical identifier physically connected to the produced active pharmaceutical ingredient and/or intermediate(s) thereof.

In one embodiment the decentral identifier relates to data associated with at least one product produced from the active pharmaceutical ingredient and/or intermediate(s) thereof, wherein the one or more environmental attribute(s) associated with the at least one product is derived from one or more environmental attribute(s) associated with the active pharmaceutical ingredient and/or intermediate(s) thereof. The one or more environmental attribute(s) associated with the active pharmaceutical ingredient and/or intermediate(s) thereof may be associated with the one or more input material(s) and/or the chemical process(s) used to produce the active pharmaceutical ingredient and/or intermediate(s) thereof. The decentral identifier may relate to any identifier uniquely associated with the active pharmaceutical ingredient and/or intermediate(s) thereof. The decentral identifier may be associated with the physical entity of the active pharmaceutical ingredient and/or intermediate(s) thereof. The decentral identifier may refer to a single batch of active pharmaceutical ingredient and/or intermediate thereof. The decentral identifier may be associated with a group of active pharmaceutical ingredients and/or intermediates thereof. The identifier may refer to multiple physical entities of the active pharmaceutical ingredient and/or intermediate(s) thereof. The decentral identifier may be associated with continuous or semi-continuous stream of active pharmaceutical ingredient and/or intermediate(s) thereof. The identifier may refer to a stream of the active pharmaceutical ingredient and/or intermediate(s) thereof e.g. over a certain time period or from a certain supplier.

In one embodiment the one or more environmental attribute(s) associated with the active pharmaceutical ingredient(s) and/or intermediate(s) thereof are provided from at least one balancing account configured to store environmental attribute(s) associated with input material(s). The balancing account may relate to storage structure associated with metadata, such as an environmental attribute type. For instance, the balancing account may be associated with metadata indicating the environmental attribute type to be recycled-content or bio-based. An inbound allocator may be configured to allocate the one or more environmental attribute(s) associated with input material(s) to at least one balancing account e.g. on entry of the input material to the chemical production network. The balancing account may be associated with the respective environmental attribute type. An outbound assigner may be configured to assign at least one environmental attribute from the at least one balancing account associated with the respective environmental attribute to the decentral identifier. One or more environmental attribute(s) may be assigned to the at least one decentral identifier. Assignment may include de-allocation of the one or more environmental attributes from the balancing account associated with the respective environmental attribute type. By using the balancing accounts environmental attributes of input materials can be reliably tracked and assigned to active pharmaceutical ingredients and/or intermediates thereof.

In one embodiment the one or more environmental attribute(s) associated with the active pharmaceutical ingredient(s) and/or intermediate(s) thereof are provided from at least one balancing account configured to store environmental attribute(s) associated with input material(s). An inbound allocator may be configured to allocate the one or more environmental attribute(s) to at least one balancing account associated with the respective environmental attribute, e.g. on input material entering the chemical production network. The balancing account may be associated with the respective environmental attribute type. The one or more environmental attribute(s) associated with the input materials may be allocated to the balancing account associated with the respective environmental attribute type. An outbound assigner may be configured to assign or link at least one environmental attribute from the at least one balancing account associated with the respective environmental attribute type to the decentral identifier. This may include de-allocation of the one or more environmental attribute(es) from the balancing account associated with the respective environmental attribute type. By using the balancing accounts environmental attributes can be tacked through the chemical production network. This way the environmental attributes may be detached from the material flow. Attribution of environmental attribute(s) may be conducted on a mass balance basis for the chemical production network. In such approach the total mass of input materials and active pharmaceutical ingredient and/or intermediate(s) thereof as well as the attribution of respective environmental attribute(s) associated with input materials and active pharmaceutical ingredient and/or intermediate(s) thereof are balanced.

In one embodiment the one or more environmental attribute(s) are associated with at least one property related to the environmental impact of the one or more input material(s) and/or the chemical process(s). The one or more environmental attribute(s) may specify environmental properties of the input material(s) used to produce the active pharmaceutical ingredient and/or intermediate(s) thereof and/or the one or more environmental attribute(s) may specify environmental properties of the chemical process(es) used to produce the active pharmaceutical ingredient and/or intermediate(s) thereof. The one or more environmental attribute(s) may be generated from environmental properties of the input material(s) used to produce the active pharmaceutical ingredient and/or intermediate(s) thereof, process data associated with the chemical processing of the input material(s) and/or energy data associated with the energy consumption of the chemical processing. The one or more environmental attribute(s) may include a recycled content associated with the input material(s) and allocated or allocatable to the active pharmaceutical ingredient and/or intermediate(s) thereof, a renewable content associated with the input material(s) and allocated or allocatable to the active pharmaceutical ingredient and/or intermediate(s) thereof, and/or a product carbon footprint associated with the active pharmaceutical ingredient and/or intermediate(s) thereof.

In one embodiment the production operating apparatus is configured to gather environmental attributes associated with the produced active pharmaceutical ingredient and/or intermediate(s) thereof before, during and/or after production of the active pharmaceutical ingredient and/or intermediate(s) thereof by the chemical production network. The environmental attributes associated with the produced active pharmaceutical ingredient and/or intermediate(s) thereof may relate to input material(s). The environmental attributes associated with input materials may be provided before, during and/or after production of the active pharmaceutical ingredient and/or intermediate(s) thereof by the chemical production network. The environmental attributes associated with input materials may be allocated to at least one balancing account before, during and/or after production of the active pharmaceutical ingredient and/or intermediate(s) thereof by the chemical production network. The environmental attributes associated with the produced active pharmaceutical ingredient and/or intermediate(s) thereof may relate to environmental properties generated from process data associated with the chemical processing of the input material(s) and/or energy data associated with the energy consumption of the chemical processing. The environmental attributes associated with the produced active pharmaceutical ingredient and/or intermediate(s) thereof may be generated before, during and/or after production of the active pharmaceutical ingredient and/or intermediate(s) thereof by the chemical production network. The process data associated with the chemical processing of the input material(s) and/or energy data associated with the energy consumption of the chemical processing may be gathered prior, during and/or after production of the active pharmaceutical ingredient and/or intermediate(s) thereof.

In one embodiment the active pharmaceutical ingredient and/or intermediate(s) thereof passport or the digital asset may include the decentral identifier associated with the active pharmaceutical ingredient and/or intermediate(s) thereof and the one or more environmental attribute(s) linked to the decentral identifier. The one or more environmental attribute(s) may be linked to the decentral identifier included in the active pharmaceutical ingredient and/ or intermediate(s) thereof passport or the digital asset. The one or more environmental attribute(s) may be stored in a data base associated with or of the active pharmaceutical ingredient and/or intermediate(s) thereof producer for access by any consumer and/or customer of the active pharmaceutical ingredient and/or intermediate(s) thereof. The one or more environmental attribute(s) may be stored in a data base associated with or of the active pharmaceutical ingredient and/ or intermediate(s) thereof producer for transfer to a consumer and/or customer of the active pharmaceutical ingredient and/or intermediate(s) thereof e.g. when accessed or on providing the active pharmaceutical ingredient and/or intermediate(s) thereof. The decentral identifier may comprise any unique identifier uniquely associated with the active pharmaceutical ingredient and/or intermediate(s) thereof producer and active pharmaceutical ingredient and/or intermediate(s) thereof data such as the environmental attributes. The decentral identifier may include a Universally Unique IDentifier (UUID) or a Digital IDentifier (DID). The decentral identifier may be issued by a central or decentral identity issuer. The decentral identifier may be linked to authentication and/or authorization information. Via the decentral identifier and its unique association with the active pharmaceutical ingredient and/or intermediate(s) thereof producer and active pharmaceutical ingredient and/or intermediate(s) thereof data, such as the environmental attributes, access to the active pharmaceutical ingredient and/or intermediate(s) thereof data may be controlled by the active pharmaceutical ingredient and/or intermediate(s) thereof producer. This contrasts with central authority schemes, where identifiers are provided by such central authority and access to data is controlled by such central authority. Decentral in this context refers to the usage of the identifier in implementation as controlled by the data owner, such as the active pharmaceutical ingredient and/or intermediate(s) thereof producer.

The decentral identifier may be uniquely associated with the active pharmaceutical ingredient and/or intermediate(s) thereof or the physical entity of the active pharmaceutical ingredient and/or intermediate(s) thereof, e.g. as packaged for transportation to the consumer and/or customer of the active pharmaceutical ingredient and/or intermediate(s) thereof. The decentral identifier may be uniquely to the one or more environmental attribute(s). The active pharmaceutical ingredient and/or intermediate(s) thereof passport or the digital asset may include one or more digital representation(s) pointing to active pharmaceutical ingredient and/or intermediate(s) thereof data including the environmental attribute(s) or parts thereof including the environmental attribute(s). The digital representation may comprise at least one interface to a data providing service. It may further include at least one interface to a data consuming service. It may include an endpoint for data exchange or sharing (resource endpoint) or an endpoint for service interaction (service endpoint), that is uniquely identified via a communication protocol. The digital representation(s) pointing to active pharmaceutical ingredient and/or intermediate(s) thereof data or parts thereof may be uniquely associated with the decentral identifier.

The active pharmaceutical ingredient and/or intermediate(s) thereof passport or the digital asset may comprise or be connected to a digital representation of active pharmaceutical ingredient and/or intermediate(s) thereof data, such as environmental attribute(s). The digital representation may include a representation for accessing the active pharmaceutical ingredient and/or intermediate(s) thereof data, such as environmental attribute(s) or part thereof. The digital representation may include a representation of active pharmaceutical ingredient and/or intermediate(s) thereof data, such as environmental attribute(s). The active pharmaceutical ingredient and/or intermediate(s) thereof passport or the digital asset may include or be connected to data related to the active pharmaceutical ingredient and/or intermediate(s) thereof data, such as environmental attribute(s), the authentication information and the decentral identifier. The data related to the active pharmaceutical ingredient and/or intermediate(s) thereof data, such as environmental attribute(s), may include the digital representation of the active pharmaceutical ingredient and/or intermediate(s) thereof data, such as environmental attribute(s).

### BRIEF DESCRIPTION OF THE DRAWINGS

In the following, the present disclosure is further described with reference to the enclosed figures:
- Fig. 1: illustrates schematically an example of a chemical production network producing one or more output material(s) from one or more input material(s) in connection with a production operating system including an attribute management system.
- Fig. 2: illustrates schematically an example of attributing environmental attributes of input materials to output materials of the chemical production network.
- Fig. 3: illustrates schematically an example of attributing environmental attributes of input materials and chemical processes to an output material of the chemical production network.
- Fig. 4: illustrates schematically another example of a method or apparatus for providing environmental attributes associated with output materials to a material user as data consumer via a decentral network.
- Fig. 5: illustrates schematically an example of a method or apparatus for providing environmental attributes of output materials across value chains via the decentral network.
- Fig. 6: illustrates schematically an example of a chemical production network for producing active pharmaceutical ingredients and/or intermediates thereof associated with the digital asset.

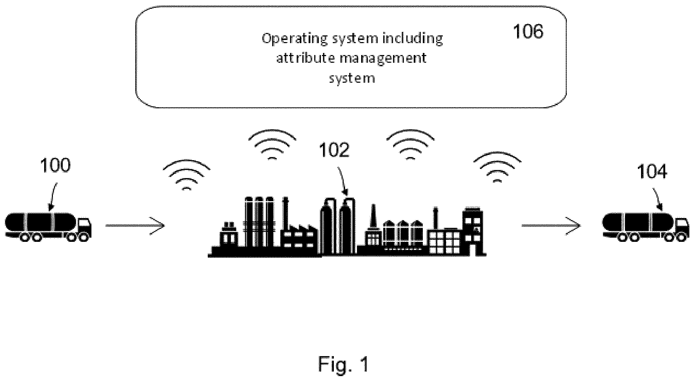

Fig. 1 illustrates an example of a chemical production network 102 producing one or more output material, in particular active pharmaceutical ingredients and/or intermediates thereof 104 from one or more input material(s) 100 in connection with a production operating system 106 including an attribute management system.

For producing one or more output material(s), in particular active pharmaceutical ingredient(s) and/or intermediate(s) thereof104 different input materials 100 may be provided as physical inputs to the chemical production network 102. The physical input material(s) 100 and output material(s), in particular active pharmaceutical ingredient(s) and/or intermediate(s) thereof, 104 may be associated with one or more properties related to environmental impact. The properties related to environmental impact may be digitalized in the form of environmental attributes such as recycled or bio-based content of the input materials. The production operating system 106 may be configured to ingest such environmental attributes and to track the environmental attributes across the chemical production network 102 from input materials 100 to output material(s), in particular active pharmaceutical ingredient(s) and/or intermediates thereof, 104.

The chemical production network 102 may include multiple interlinked processing steps. The chemical production network 102 may be an integrated chemical production network 102 with interrelated production chains. The chemical production network 102 may include multiple different production chains that have at least one intermediate product in common. The chemical production network 102 may include multiple stages of the chemical value chain. The chemical production network 102 may include the producing, refining, processing and/or purification of gas or crude oil. The chemical production network 102 may include a stream cracker, or a syngas plant connected to multiple production chains that output products 104 from the effluent of such plants. The chemical production network 102 may include multiple production chains that output from one or more input material(s) 100 one or more output material(s), in particular active pharmaceutical ingredient(s) and/or intermediate(s) thereof, 104. The chemical production network 102 may include multiple tiers of a chemical value chain. The chemical production network 102 may include a physically interconnected arrangement of production sites. The production sites may be at the same location or at different locations. In the latter case the production sites may be interconnected by means of dedicated transportation systems such as pipelines, supply chain vehicles, like trucks, supply chain ships or other cargo transportation means.

The chemical production network 102 may chemically convert input materials 100 to one or more output material(s), in particular active pharmaceutical ingredient(s) and/or intermediate(s) thereof, 104. The chemical production network 102 may convert input materials 100 by way of chemical conversion to one or more output material(s), in particular active pharmaceutical ingredient(s) and/or intermediate(s) thereof, 104.

The input materials 100 may be fed to the chemical production network 102 at any entry point. The input materials 100 may be fed to the chemical production network 102 at the start of the chemical production network 102. Input materials 100 may for example make up the feedstock of a steam cracker. The input material 100 may include non-fossil input material, such as bio-based or recycled material, and/or fossil input material for the manufacture of chemical intermediates and chemical output material(s), in particular active pharmaceutical ingredient(s) and/ or intermediate(s) thereof, 104.

The chemical production network 102 may include multiple production steps. The production steps included in the chemical production network 102 may be defined by the system boundary of the chemical production network 102. The system boundary may be defined by location or control over production processes. The system boundary may be defined by the site of the chemical production network 102. The system boundary may be defined by production processes controlled by one entity or multiple entities jointly. The system boundary may be defined by value chain with staggered production processes to an end product, which may be controlled by multiple entities separately. The chemical production network 102 may include a waste collection and sorting step, a recycling step such as pyrolysis, a cracking step such as steam cracking, a separation step to separate intermediates of one process step and further processing steps to convert such intermediates to output material(s), in particular active pharmaceutical ingredient(s) and/or intermediate(s) thereof, 104 leaving the system boundary of the chemical production network 102.

The production operating system 106 of the chemical production network 102 may be configured to monitor and/or control the chemical production network 102 based on operating parameters of the different processes. One process step monitored and/or controlled may be the feed of input materials 100 or the release of output material(s), in particular active pharmaceutical ingredient(s) and/or intermediate(s) thereof, 104. Another process step monitored and/or controlled may be the registration of environmental attributes associated with input materials 100 entering the system boundary of the chemical production network 102. Yet another process step monitored and/or controlled may be the attribution of environmental attributes to output material(s), in particular active pharmaceutical ingredient(s) and/or intermediate(s) thereof, 104 produced via the chemical production network 102. Yet another process step monitored and/or controlled may be the management of environmental attributes associated with input materials 100 and output material(s), in particular active pharmaceutical ingredient(s) and/or intermediate(s) thereof, 104 of the chemical production network 102.

The production operating system 106 may be configured to register inbound environmental attributes and to assign outbound environmental attributes. The production operating system 106 may be configured to access data related the inputs materials 100, the processes and/or the output material(s), in particular active pharmaceutical ingredient(s) and/or intermediate(s) thereof, 104 used in the chemical production network 102. For example, the production operating system 106 may be configured to register a recycled or bio-based content of the one or more input material(s) 100 used in the chemical production network 102 as environmental attribute. The production operating system 106 may be configured to allocate the environmental attribute to at least one balancing account associated with the recycled or bio-based content of the input materials 100. The production operating system 106 may be configured to allocate at least a part of the environmental attributes from the at least one balancing account to the at least one output material(s), in particular to the active pharmaceutical ingredient(s) and/or intermediate(s) thereof, 104.

The production operating system 102 may be configured to handle environmental attributes related to the input materials 100 and output material(s), in particular active pharmaceutical ingredient(s) and/or intermediate(s) thereof, 104 of the chemical production network 102. For example, the production operating system 106 may be configured to determine environmental attributes associated with the use of input materials 100 impacting the environmental property of the chemical production network 102 and the output material(s), in particular active pharmaceutical ingredient(s) and/or intermediate(s) thereof 104 produced by the chemical production network 102. Further in particular, the production operating system 102 may be configured to determine environmental attributes associated with the output material(s), in particular active pharmaceutical ingredient(s) and/or intermediate(s) thereof 104. This way the production operating system 102 may be configured to store environmental attributes in balancing accounts or to withdraw environmental attributes from the balancing accounts. The environmental attributes may hence be viewed as a credit that may be deposited in an account or deducted from an account related to the input and output material(s), in particular active pharmaceutical ingredient(s) and/or intermediate(s) thereof, of the chemical production network 102. This way the environmental impact of the production may be tracked and/or traced.

In chemical production networks 102 multiple value chains may be linked. Additionally different input materials 100 or chemical processes impacting the environmental property of output material(s), in particular active pharmaceutical ingredient(s) and/or intermediate(s) thereof, 104 produced by the chemical production network 102 may be used. Examples of input materials 100 impacting at least one environmental property of output material(s), in particular active pharmaceutical ingredient(s) and/or intermediates thereof 104 produced from such input materials 100 are recycled, renewable or bio-based input materials 104. Examples of chemical processes impacting the environmental property include chemical processes using environmentally friendly technology such as carbon capture, carbon utilization or heat pumps.

Owing to the processing of chemicals in continuous or semi-continuous production and the complexity of chemical production networks 102, traceability of the input materials through the network may be hampered. In such scenarios, an equivalent environmental attribute signifying the impact on the environmental property of output material(s), in particular active pharmaceutical ingredient(s) and/or intermediate(s) thereof 104 produced by the chemical production network may be allocated to balancing accounts and assigned to one or more output material(s), in particular active pharmaceutical ingredient(s) and/ or intermediate(s) thereof, 104 of the chemical production network 102. The environmental attributes may hence be decoupled from the physical material flow inside the chemical production network 102. Decoupling may be based on the mass balance model in that the equivalent amount assigned to the one or more output material(s), in particular active pharmaceutical ingredient(s) and/or intermediate(s) thereof may not exceed the equivalent amount provided by input materials or processes. If an equivalent amount has been allocated to the virtual account of one environmental attribute type, it may not be allocated a second time to another virtual account of the one environmental attribute type. Environmental attribute types may be recycled, bio-based, renewable or the like. Environmental attributes may be provided in the form of digital assets or active pharmaceutical ingredient(s) and/or intermediate(s) thereof passports attached to the physical entity of the active pharmaceutical ingredient and/or intermediate(s) thereof.

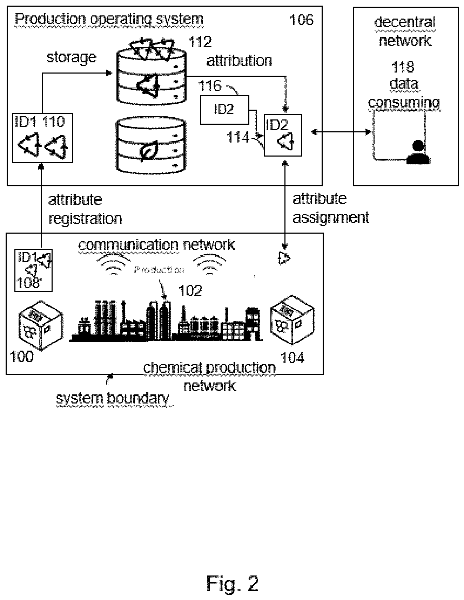

Fig. 2 illustrates schematically an example of attributing environmental attributes associated with input materials 100 to output material(s), in particular active pharmaceutical ingredient(s) and/or intermediate(s) thereof 104 of the chemical production network 102.

As shown in Fig. 1 the chemical production network 102 and operations of the chemical production network 102 may be monitored and/or controlled by a production operating system 106. The production operating system 106 may be configured to track environmental attributes from input materials 100 fed to the chemical production network 102 to output material(s), in particular active pharmaceutical ingredient(s) and/or intermediate(s) thereof, 104 produced by the chemical production network 102. For tracking the operating system 106 may be configured to register environmental attributes associated with the input materials 100 provided to the chemical production network 102 and to attribute environmental attributes to output material(s), in particular active pharmaceutical ingredient(s) and/or intermediate(s) thereof, 104 produced by the chemical production network 102.

The input materials 100 such as pyrolysis oil, bio-naphtha or biogas may be provided to the chemical production network 102. The input materials 100 may enter the chemical production network 104 at the entry point, such as a such as a steam cracker or a syngas plant. The input materials 100 may be used in the chemical production network 102 to produce one or more output material(s), in particular active pharmaceutical ingredient(s) and/or intermediate(s) thereof, 104 from the input materials 100. output material(s), in particular active pharmaceutical ingredient(s) and/or intermediate(s) thereof, 104 may be provided at exit points of the chemical production network 102. Further output material(s) may be MDI, TDI, PA6, EPS, PC, Polyols, Caprolactam, adipic acid, HMD, Polyamides.

On entry of the input material 100, input material data 108 may be provided via a communication network to a computing interface of the production operating system 106. A data provider, such as a QR code reader, may be configured to provide material data 108 related to the one or more input material(s) 100 and respective environmental attributes 108 to a computing interface configured to allocate the environmental attributes associated with the input materials 100. The material data 108 may include the input material identifier and environmental attributes associated with the input materials 100. The input material identifier may be associated with the physical entity of the input material 100 entering the chemical production network 102. The material data may be provided on, prior or after providing of the one or more input material(s) at entry points to the chemical production network 102.

The input material identifier may be linked to the environmental attribute(s) associated with the respective input material(s) 100, the amount of input material 100 and the certificate certifying the environmental attribute(s). The amount of input material may be a measured amount of input material 100 fed to a plant or storage of the chemical production network 102 for producing one or more output material(s), in particular active pharmaceutical ingredient(s) and/or intermediate(s) thereof 104 from the input material(s) 100. The input material identifier associated with the respective input material 100, the environmental attribute(s) associated with the respective input material(s) 100 and the amount of input material(s) 100 provided to the chemical production network 102 may be provided to the production operating system 106. Such data may be provided via a communication network on entry to chemical production network 102, or the data may be transferred from a computing system to the production operating system 106.

An inbound allocator 110 may be configured to allocate the one or more environmental attribute(s) to at least one balancing account 112 associated with the respective environmental attribute. For example, one balancing account 112 may relate to environmental attributes from recycled material and another balancing account 112 may relate to environmental attributes from bio-based material. The balancing account may be associated with the respective environmental attribute type, such as bio-based or recycled. Based on such association the balancing account associated with the environmental attribute type of the respective input material 100 may be selected. The environmental attributes may be allocated to the selected balancing account. For example, the account 112 for recycled material may be selected and the environmental attribute may be allocated to such account 122.

To allocate, the one or more environmental attribute(s) may be converted to balancing units and the balancing units may be allocated to the balancing account 122. The conversion may be based on a conversion factor such as mass, weight, carbon atoms, hydrogen atoms, methane equivalents or any other suitable measure for quantifying the environmental impact of the environmental attribute. The conversion factor may hence take into account the difference between producing chemical products from conventional input material(s) and producing chemical products from non-conventional input material(s) or producing chemical products from a mix of conventional and non-conventional input materials. The conversion factor may relate to differences in chemical and/or physical properties of conventional and non-conventional input material(s).

By using the balancing accounts 112 it can be ensured that environmental attributes of input materials 100 are only used once for assignment to active pharmaceutical ingredients and/or intermediates thereof 104. This way double counting on input or output is avoided to ensure positive environmental impact can be reliable tracked and assigned to active pharmaceutical ingredients and/or intermediates thereof 104.

An identifier provider 116 may be configured to provide the active pharmaceutical ingredient and/or intermediate thereof identifier associated with the active pharmaceutical ingredient and/or intermediate(s) thereof produced by the chemical production network 102 and provided at the exit point from the chemical production network 102.

An outbound assigner 114 may be configured to assign at least one environmental attribute from the at least one balancing account 112 associated with the respective environmental attribute to the active pharmaceutical ingredient and/ or intermediate(s) thereof identifier ID2. One or more environmental attribute(s) may be assigned to the at least one active pharmaceutical ingredient and/or intermediate(s) thereof identifier ID2. Assignment may include de-allocation of the one or more environmental attributes from the balancing account 112 associated with the respective environmental attribute type. Assignment may include converting one or more balancing unit(s) to one or more environmental attribute(s).

Assigning at least one environmental attribute associated with input material(s) to active pharmaceutical ingredient(s) and/or intermediate(s) thereof may include the linking of the active pharmaceutical ingredient and/or intermediate(s) thereof identifier ID2 with the environmental attribute. The active pharmaceutical ingredient and/or intermediate(s) thereof identifier ID2 may be associated with the physical entity of the active pharmaceutical ingredient and/or intermediate(s) thereof. This way the virtual identifier of a material may be uniquely linked to the physical material. Such linking may include a physical or virtual link of identifiers uniquely associated with the physical material. For physical linking a tag or code may be physically connected to the material, e.g., by printing a QR code on the packaging. For virtual linking different identifiers associated with the physical material may be linked. For example, an order number, a batch number, LOT number or a combination thereof may be linked.

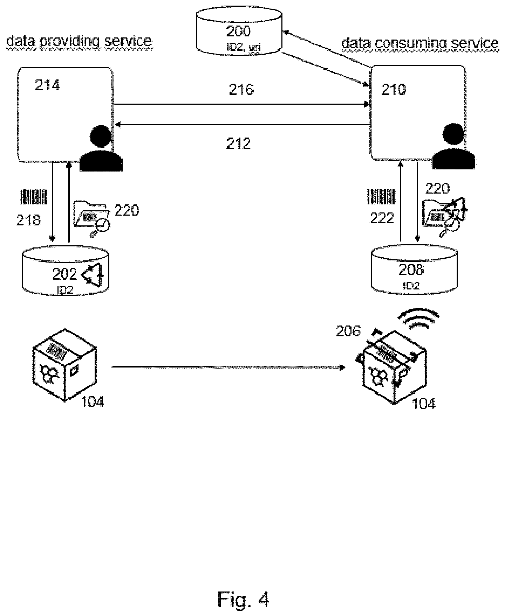

The outbound assigner 114 may be configured to provide the environmental attributes associated with the active pharmaceutical ingredient(s) and/or intermediate(s) thereof to a data consumer, such as a system associated with a user of the active pharmaceutical ingredient(s) and/or intermediate(s) thereof. The outbound assigner 114 may be configured to provide the environmental attributes associated with the active pharmaceutical ingredient(s) and/or intermediate(s) thereof to a decentral network as will be described in the example of Fig. 4. Environmental attributes may be provided via the above ID based schema in the form of digital assets or active pharmaceutical ingredient and/or intermediate(s) thereof passports associated with the physical entity of the active pharmaceutical ingredients and/or intermediates thereof.

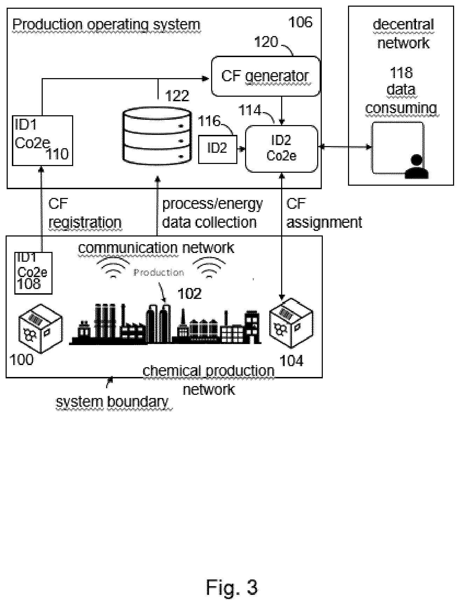

Fig. 3 illustrates schematically an example of attributing environmental attributes of input materials 100 and chemical processes to the active pharmaceutical ingredient and/or intermediate(s) thereof 104 of the chemical production network 102.

As described in the context of Figs. 1 and 2 the chemical production network 102 and operations of the chemical production network 102 may be monitored and/or controlled by a production operating system 106. Input materials 100 such as pyrolysis oil, bio-naphtha or biogas may be provided to the chemical production network 102. The input materials 100 may be used in the chemical production network 102 to produce one or more active pharmaceutical ingredient(s) and/or intermediate(s) thereof 104 from the input materials 100.

On entry of the input material 100, input material data 108 may be provided via a communication network to a computing interface of the production operating system 106. A data provider, such as a QR code reader, may be configured to provide material data 108 related to the one or more input material(s) 100 and respective environmental attributes 108 to a computing interface configured to allocate the environmental attributes associated with the input materials 100. The material data 108 may include the input material identifier and environmental attributes associated with the input materials 100. The input material identifier may be associated with the physical entity of the input material 100 entering the chemical production network 102. The input material identifier may be linked to the carbon footprint of the input material 100 as environmental attribute. The material data may be provided on, prior or after providing of the one or more input material(s) at entry points to the chemical production network 102.

The inbound allocator 110 may be configured to retrieve the one or more environmental attribute(s) and to provide such attributes to the carbon footprint (CF) generator 120. A process data provider 122 may be configured to gather process data associated with the chemical processing of the input material(s) 100 to produce the active pharmaceutical ingredient(s) and/or intermediate(s) thereof 104. The process data provider 122 may be configured to gather energy data associated with the energy consumption of the chemical processing. The process data provider 122 may be configured to provide the process data and the energy data to the CF generator 120.

The CF generator 120 may be configured to determine the carbon footprint of the active pharmaceutical ingredient and/or intermediate(s) thereof produced by the chemical production network. The carbon footprint of the of the active pharmaceutical ingredient and/ or intermediate(s) thereof may be determined based on the process data, the energy data and the carbon footprint of the input material(s) 100 used to produce the active pharmaceutical ingredient and/or intermediate(s) thereof.

An identifier provider 116 may be configured to provide the active pharmaceutical ingredient and/or intermediate(s) thereof identifier associated with the active pharmaceutical ingredient and/or intermediate(s) thereof produced by the chemical production network 102 and provided at the exit point from the chemical production network 102.

An outbound assigner 114 may be configured to assign the determined carbon footprint to the active pharmaceutical ingredient and/or intermediates thereof identifier ID2. One or more environmental attribute(s) may be assigned to the at least one active pharmaceutical ingredient and/or intermediate(s) thereof identifier ID2, such as described in the context of Fig. 2.

The outbound assigner 114 may be configured to provide the environmental attributes, in particular the carbon footprint, associated with the active pharmaceutical ingredient and/or intermediate(s) thereof to a data consumer, such as a system associated with a user of the active pharmaceutical ingredient and/or intermediate(s) thereof. The outbound assigner 114 may be configured to provide the environmental attributes associated with the active pharmaceutical ingredient and/or intermediate(s) thereof to a decentral network as will be described in the example of Fig. 4. Environmental attributes may be provided via the above ID based schema in the form of digital assets or active pharmaceutical ingredient and/or intermediate(s) thereof passports associated with the physical entity of the active pharmaceutical ingredients and/or intermediates thereof.

Fig. 4 illustrates schematically an example of a method or apparatus for providing environmental attributes associated with active pharmaceutical ingredient and/or intermediates thereof to a material user as data consumer via a decentral network.

The active pharmaceutical ingredient and/or intermediate(s) thereof 104 as produced by the chemical production network 102 may be provided in association with the digital asset as described in the context of Figs. 2 and 3. The digital asset may include the active pharmaceutical ingredient and/or intermediate(s) thereof identifier. The digital asset may include one or more environmental attribute(s) such as the product carbon footprint, recycled content or bio-based content. The digital asset may relate to one or more environmental attribute(s) such as the product carbon footprint, recycled content or bio-based content. The digital asset may include a digital representation of one or more environmental attribute(s) such as the product carbon footprint, recycled content or bio-based content.

The digital asset may further include or relate to authentication and/or authorization information linked to the active pharmaceutical ingredient and/or intermediate(s) thereof identifier. The authentication and/or authorization information may be provided for authentication and/or authorization of a data providing service 208 and/or data consuming service 210. The active pharmaceutical ingredient and/or intermediate(s) thereof identifier may include or relate to a decentral identifier, that is uniquely associated with the active pharmaceutical ingredient and/or intermediate(s) thereof. The decentral identifier may be connected to the digital representation of the environmental attributes. The digital representation may include a representation for accessing the environmental attributes or parts thereof. The decentral identifier may include a Universally Unique IDentifier (UUID) or a Digital IDentifier (DID). The decentral identifier may include any unique identifier uniquely associated with a data owner and/or active pharmaceutical ingredient and/or intermediate(s) thereof. The data owner may be the producer of the active pharmaceutical ingredient and/or intermediate(s) thereof. Via the decentral identifier and its unique association with the data owner and/or active pharmaceutical ingredient and/or intermediate(s) thereof access to the material configuration data may be controlled by the data owner.

The digital asset including the digital representation of one or more environmental attribute(s) such as the product carbon footprint, recycled content or bio-based content may be stored in a decentral data base 200. The one or more environmental attribute(s) such as the product carbon footprint, recycled content or bio-based content may be stored in a data base 202 associated with the data owner, such as the producer of the active pharmaceutical ingredient and/or intermediate(s) thereof 104.

The active pharmaceutical ingredient and/or intermediate(s) thereof 104 may be physically delivered to a user of the active pharmaceutical ingredient and/or intermediate(s) thereof. The active pharmaceutical ingredient and/or intermediate(s) thereof may be connected with a QR-code having encoded the active pharmaceutical ingredient and/or intermediate(s) thereof identifier.

The user of the active pharmaceutical ingredient and/or intermediate(s) thereof may read the QR-code through a QR-code reader 206. The active pharmaceutical ingredient and/or intermediate(s) thereof identifier may be provided to a data base 208 associated with the consumer or customer of the active pharmaceutical ingredient and/ or intermediate(s) thereof 104. In other embodiments the consumer/customer of the active pharmaceutical ingredient and/or intermediate(s) thereof may retrieve the active pharmaceutical ingredient and/or intermediate(s) thereof identifier through the decentral data base 200.

The data owner in this example may be the input material producer, the output material producer, the output material user, the end product producer. The data owner may comprise any entity generating data. The data generating node may be coupled to the data owner or the entity owning or producing physical products from or for which data is generated. The data may be generated by a third-party entity on behalf of the entity owning physical products from or for which data is generated.

The data consuming service 210 may comprise computer-executable instructions for accessing and/or processing data, such as active pharmaceutical ingredient and/or intermediate(s) thereof data, associated with the data owner. The data providing service 210 may comprise computer-executable instructions for providing and/or processing data, such as active pharmaceutical ingredient and/or intermediate(s) thereof data, associated with the data owner for accessing and/or processing by the data consuming service 214.

Based on the received active pharmaceutical ingredient and/or intermediates thereof identifier a request to access the environmental attributes associated with the active pharmaceutical ingredient and/or intermediates thereof identifier may be triggered by the data consuming service 210 as signified by arrow 212. The active pharmaceutical ingredient and/or intermediate(s) thereof identifier may be provided to the data providing service 214 associated with or of the producer of the active pharmaceutical ingredient and/or intermediate(s) thereof 104. In addition, authentication and/or authorization information may be provided.

The request may be authenticated and/or authorized to access the environmental attributes associated with the active pharmaceutical ingredient and/or intermediate(s) thereof identifier. Based on successful authorization and/or authentication access to the environmental attributes associated with the active pharmaceutical ingredient and/or intermediate(s) thereof identifier may be granted.

For access the active pharmaceutical ingredient and/or intermediate(s) thereof identifier may be provided to the data providing service 214 as signified by arrow 212. The data providing service 214 may use the received active pharmaceutical ingredient and/or intermediate(s) thereof identifier to retrieve the environmental attributes associated with the active pharmaceutical ingredient and/ or intermediate(s) thereof 104 as signified by arrows 218 and 220. The environmental attributes associated with the active pharmaceutical ingredient and/or intermediate(s) thereof 104 provided to the data providing service 214 may be provided to the data consuming service 210 as signified by arrow 216. The environmental attributes associated with the active pharmaceutical ingredient and/or intermediate(s) thereof 104 may be stored in the data base 208 associated with the user of the active pharmaceutical ingredient and/or intermediate(s) thereof 104 as signified by arrow 220.

Through the output identifier or decentral identifier, the environmental attributes can be uniquely associated with the active pharmaceutical ingredient and/or intermediate(s) thereof. Through the decentral network the environmental attributes may be transferred between the producer of the active pharmaceutical ingredient and/or intermediate(s) thereof and the user of the active pharmaceutical ingredient and/or intermediate(s) thereof. This way the environmental attributes can be shared with unique association to the active pharmaceutical ingredient and/or intermediate(s) thereof and without central intermediary directly between the value chain players. This allows for transparency of environmental attributes across the value chain and positive environmental impacts from active pharmaceutical ingredient(s) and/or intermediate(s) thereof produced by the chemical production network 102 can be tracked through the value chain.

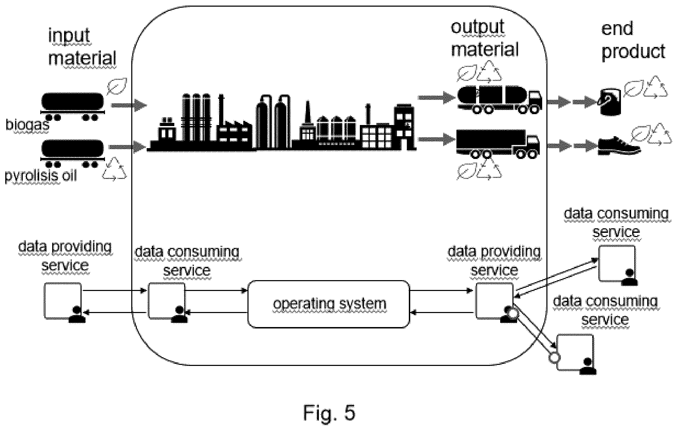

Fig. 5 illustrates schematically an example of a method or apparatus for providing environmental attributes associated with active pharmaceutical ingredient and/or intermediate(s) thereof across value chains via the decentral network.

In the example of Fig. 5 a fully connected value chain including the chemical production network is illustrated. In the example, the input material provider, the active pharmaceutical ingredient and/or intermediate(s) thereof producer, the active pharmaceutical ingredient and/or intermediate(s) thereof user and the end product producer may be connected to the decentral network as described in the context of Fig. 4. Environmental attributes may be provided via the ID based schema described in the context of Figs. 2-4 in the form of digital assets or active pharmaceutical ingredient and/or intermediate(s) thereof passports associated with the physical entity of the input material, the active pharmaceutical ingredient and/ or intermediate(s) thereof, any intermediate product or the end product.

The input material provider may provide the input materials such as biogas or pyrolysis oil. The environmental attributes of the input material may be provided through the data providing service connected to the decentral network as described in the context of Fig. 4. The active pharmaceutical ingredient and/or intermediate(s) thereof producer may produce the active pharmaceutical ingredient and/or intermediate(s) thereof from the input material(s) provided to the chemical production network. The active pharmaceutical ingredient and/or intermediate(s) thereof producer may access the environmental attributes associated with the input material through a data consuming service connected to the decentral network as described in the context of Fig. 4. The active pharmaceutical ingredient and/or intermediate(s) thereof producer may manage the environmental attributes via the production operating system as described in the context of Figs. 1 to 3. The active pharmaceutical ingredient and/or intermediate(s) thereof producer may assign the environmental attributes associated with the input materials or environmental attributes associated with the chemical production network such as the carbon footprint, to the active pharmaceutical ingredient and/or intermediate(s) thereof as described in the context of Figs. 1 to 3. The active pharmaceutical ingredient and/or intermediate(s) thereof producer may provide the environmental attributes associated with the active pharmaceutical ingredient and/or intermediate(s) thereof through the data providing service connected to the decentral network as described in the context of Fig. 4. The active pharmaceutical ingredient and/or intermediate(s) thereof user or the end product producer may access the environmental attributes associated with the active pharmaceutical ingredient and/or intermediate(s) thereof through the data consuming service connected to the decentral network as described in the context of Fig. 4.

The respective data owners in this example may be the input material producer, the output material producer, the output material user, the end product producer. The data owner may comprise any entity generating data. The data generating node may be coupled to the data owner or the entity owning or producing physical products from or for which data is generated. The data may be generated by a third-party entity on behalf of the entity owning physical products from or for which data is generated.

The data consuming service may comprise computer-executable instructions for accessing and/or processing data, such as active pharmaceutical ingredient and/or intermediate(s) thereof data, associated with the data owner. The data providing service may comprise computer-executable instructions for providing and/or processing data, such as active pharmaceutical ingredient and/or intermediate(s) thereof data, associated with the data owner for accessing and/or processing by the data consuming service.

In the example of Fig. 5 the decentral identifier may relate to the end product. Such decentral identifier may be provided to the value chain participants. Via the end product specific decentral identifier data associated with the end product produced from the active pharmaceutical ingredient and/or intermediate(s) thereof may be gathered across the production chain and assigned to the end product specific decentral identifier. For example, the one or more environmental attribute(s) associated with the end product may be derived from the environmental attribute(s) associated with the active pharmaceutical ingredient and/or intermediate(s) thereof, the input material or any other product entity present in the value chain of the end product.

This way the environmental attributes of input materials, active pharmaceutical ingredients and/or intermediates thereof and any products produced from active pharmaceutical ingredients and/ or intermediates thereof may be tracked through the value chain up to the end product. By tracking the environmental attributes of materials in such way the information can be made transparent across the value chain while the information flow can be controlled by the participants in the supply chain. In addition, the environmental attributes can be handled according to the individual participants needs by production operating systems as described in the context of Figs. 1 to 3. Overall, such tracking enables tracking of positive environmental impact by individual supply chain participants, which makes positive environmental impacts transparent and attributable to individual supply chain participants.

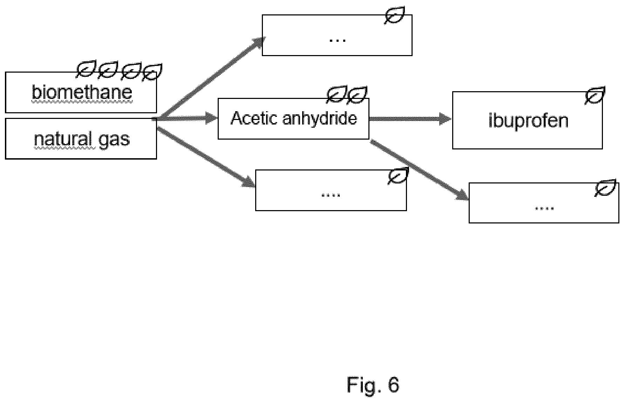

Fig. 6 illustrates schematically an example of a chemical production network for producing active pharmaceutical ingredients and/or intermediates thereof, e.g. ibuprofen, associated with the digital asset. The example of Fig. 6 is an illustrative example and should not be considered limiting.

In the example of Fig. 6 natural gas and/or biomethane may be provided to the chemical production network for producing as an example ibuprofen. Natural gas and biomethane may be the input materials in this example as mixed feedstock. From natural gas and biomethane acetic anhydride and other intermediate materials may be produced. From acetic anhydride ibuprofen and other products, such as intermediate materials or output materials, may be produced.

In the example of Fig. 6 the bio-based content of biomethane as input material may be tracked. As described in the context of Fig. 2, the biomethane content at the start of the ibuprofen production chain may be registered via the production operating system 106. Here the biomethane content may be allocated to the balancing account 112 for bio-based materials. The environmental attribute of biomethane content is hence detached from the mass flows of the chemical processing in the chemical production network 102 as illustrated in Fig. 6.

For attribution of the biomethane content used to produce the ibuprofen, the biomethane content may be determined. The biomethane content attributable to ibuprofen production may be based on mass conservation attributable to the produced ibuprofen. For example, only half of the biomethane content may be attributable to the ibuprofen and the other half may be attributable to other output products resulting from the biomethane as input material. The environmental attribute biomethane content may be attributed to such extend to the ibuprofen.

In the system shown in Fig. 2, the environmental attribute associated with the production of active pharmaceutical ingredient and/or intermediate(s) thereof may be attributed to the produced active pharmaceutical ingredient and/or intermediate(s) thereof by associating the decentral ID to the active pharmaceutical ingredient and/or intermediate(s) thereof and assigning the environmental attribute to the decentral ID. By linking the ID and the environmental attribute, the environmental attribute may be uniquely linked to the produced active pharmaceutical ingredient and/or intermediate(s) thereof. The active pharmaceutical ingredient and/or intermediate(s) thereof may be delivered to the active pharmaceutical ingredient and/or intermediate(s) thereof user. The packaging such as lose bags with active pharmaceutical ingredient and/or intermediate(s) thereof may include a QR-code. The decentral ID may be included or encoded to the QR code. This way the active pharmaceutical ingredient and/or intermediate(s) thereof user may access the environmental attributes associated to the active pharmaceutical ingredient and/or intermediate(s) thereof via the ID based protocol described in the context of Figs. 4 and 5.

Similarly to this example the chemical production network for producing active pharmaceutical ingredient(s) and/or intermediate(s) thereof associated with the digital asset may be based on the method illustrated in Fig. 3 for carbon footprints.

The present disclosure has been described in conjunction with preferred embodiments and examples as well. However, other variations can be understood and effected by those persons skilled in the art and practicing the claimed invention, from the studies of the drawings, this disclosure and the claims.

Any steps presented herein can be performed in any order. The methods disclosed herein are not limited to a specific order of these steps. It is also not required that the different steps are performed at a certain place or in a certain computing node of a distributed system, i.e. each of the steps may be performed at different computing nodes using different equipment/data processing.

As used herein "determining" also includes "initiating or causing to determine", "generating" also includes "initiating and/or causing to generate" and "providing" also includes "initiating or causing to determine, generate, select, send and/or receive". "initiating or causing to perform an action" includes any processing signal that triggers a computing node or device to perform the respective action.

In the claims as well as in the description the word "comprising" does not exclude other elements or steps and the indefinite article "a" or "an" does not exclude a plurality. A single element or other unit may fulfill the functions of several entities or items recited in the claims. The mere fact that certain measures are recited in the mutual different dependent claims does not indicate that a combination of these measures cannot be used in an advantageous implementation.

Any disclosure and embodiments described herein relate to the methods, the systems, devices, the computer program element lined out above and vice versa. Advantageously, the benefits provided by any of the embodiments and examples equally apply to all other embodiments and examples and vice versa.

All terms and definitions used herein are understood broadly and have their general meaning.

## Claims

1. A system for producing an active pharmaceutical ingredient and/or intermediate(s) thereof associated with a digital asset, the system comprising:
- a chemical production network configured to produce the active pharmaceutical ingredient and/or intermediate(s) thereof wherein the active pharmaceutical ingredient and/or intermediate(s) thereof is produced from one or more input material(s) through one or more chemical process(s) of the chemical production network, wherein the one or more input material(s) and/or the one or more chemical process(s) are associated with environmental attribute(s);
- a production operating apparatus configured to generate the digital asset by providing a decentral identifier associated with the produced active pharmaceutical ingredient and/or intermediate(s) thereof and one or more environmental attribute(s) of the one or more input material(s) and/or the one or more chemical process(es);
linking the decentral identifier and the environmental attribute(s).

2. The system of claim 1, wherein the active pharmaceutical ingredient and/or intermediate(s) thereof associated with the digital asset is selected from azelaic acid, dexpanthenol, poly[(2-oxopyrrolidin-1yl)ethylene]iodine, L-menthol, ibuprofen, ibuprofen sodium dihydrate, racemic ibuprofen lysinate, 2-hydroxyacetophenone, 4-hydroxyacetophenone.

3. The system of any of claims 1 or 2, wherein the digital asset of the active pharmaceutical ingredient and/or intermediate(s) thereof includes mass balanced environmental attributes related to the input material(s).

4. The system of any of claims 1 to 3, wherein the one or more environmental attribute(s) associated with the active pharmaceutical ingredient and/or intermediate(s) thereof are provided from at least one balancing account configured to store environmental attribute(s) associated with input material(s).

5. The system of any of claims 1 to 4, wherein the one or more environmental attribute(s) are associated with at least one property related to the environmental impact of the one or more input material(s) and/or the chemical process(s).

6. The system of any of claims 1 to 5, wherein the production operating apparatus is configured to gather environmental attributes associated with the produced active pharmaceutical ingredient and/or intermediate(s) thereof before, during and/or after production of the active pharmaceutical ingredient and/or intermediate(s) thereof by the chemical production network.

7. The system of any of claims 1 to 6, wherein the environmental attribute(s) associated with the active pharmaceutical ingredient and/or intermediate(s) thereof produced through chemical processes from one or more input material(s) provided to the chemical production network include the environmental attribute(s) associated with the input material(s), the chemical process(es) and/or the chemical production network(s).

8. The system of any of claims 1 to 7, wherein the environmental attribute(s) associated with input material(s) are provided before, during and/or after production of the active pharmaceutical ingredient and/or intermediate(s) thereof by the chemical production network, wherein the environmental attribute(s) associated with input material(s) are allocated to at least one balancing account before, during and/or after production of the active pharmaceutical ingredient and/or intermediate(s) thereof by the chemical production network.

9. The system of any of claims 1 to 8, wherein the environmental attribute(s) associated with the produced active pharmaceutical ingredient and/or intermediate(s) thereof relate to environmental properties generated from process data associated with the chemical processing of the input material(s) and/or energy data associated with the energy consumption of the chemical processing, wherein the environmental attribute(s) associated with the produced active pharmaceutical ingredient and/or intermediate(s) thereof are generated before, during and/or after production of the active pharmaceutical ingredient and/or intermediate(s) thereof by the chemical production network.

10. A method for producing an active pharmaceutical ingredient and/or intermediate(s) thereof associated with a digital asset, wherein the method comprises:
- producing the active pharmaceutical ingredient and/or intermediate(s) thereof from one or more input material(s) through one or more chemical process(s) of a chemical production network, wherein the one or more input material(s) and/or the one or more chemical process(s) are associated with environmental attribute(s);
- generating the digital asset by
providing a decentral identifier associated with the produced active pharmaceutical ingredient and/or intermediate(s) thereof and one or more environmental attribute(s) associated with the one or more input material(s) and/or the one or more chemical process(es); linking the decentral identifier and the one or more environmental attribute(s);
- providing the produced active pharmaceutical ingredient and/or intermediate(s) thereof in association with the digital asset.

11. An active pharmaceutical ingredient and/or intermediate(s) thereof associated with a digital asset including a decentral identifier associated with the active pharmaceutical ingredient and/or intermediate(s) thereof and linked to one or more environmental attribute(s) of the one or more input material(s) and/or the one or more chemical process(s) used to produce the active pharmaceutical ingredient and/or intermediate(s) thereof.

12. The active pharmaceutical ingredient and/or intermediate(s) thereof of claim 11, wherein the active pharmaceutical ingredient and/or intermediate(s) thereof is selected from, azelaic acid, dexpanthenol, poly[(2-oxopyrrolidin-1yl)ethylene]iodine, L-menthol, ibuprofen, ibuprofen sodium dihydrate, racemic ibuprofen lysinate, 2-hydroxyacetophenone, 4-hydroxyacetophenone 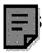

13. A method for generating a digital asset associated with an active pharmaceutical ingredient and/or intermediate(s) thereof, wherein the active pharmaceutical ingredient and/or intermediate(s) thereof is produced from one or more input material(s) through one or more chemical process(s) of a chemical production network, wherein the one or more input material(s) and/or the one or more chemical process(s) are associated with environmental attribute(s), the method comprising:
- providing a decentral identifier associated with the produced active pharmaceutical ingredient and/or intermediate(s) thereof and one or more environmental attribute(s) of the one or more input material(s) and/or the one or more chemical process(s) used to produce the active pharmaceutical ingredient and/or intermediate(s) thereof;
- linking the decentral identifier and the environmental attribute(s);
- providing the digital asset in association with the produced active pharmaceutical ingredient and/or intermediate(s) thereof, wherein the environmental attribute(s) associated with the active pharmaceutical ingredient and/or intermediate(s) thereof is made accessible to a user of the active pharmaceutical ingredient and/or intermediate(s) thereof through the digital asset.

14. A digital asset as generated according to the method of claim 13.

15. A method for using the digital asset generated according to the method of claim 13 in production of a product produced from the active pharmaceutical ingredient and/or intermediate(s) thereof associated with the digital asset or a method for using the active pharmaceutical ingredient and/or intermediate(s) thereof of any of claims 11 or 12 associated with the digital asset for producing a product from the active pharmaceutical ingredient and/or intermediate(s) thereof and deriving a digital asset associated with the product from the active pharmaceutical ingredient and/ or intermediate(s) thereof digital asset.
